# EUROPEAN PATENT APPLICATION

(11) **EP 2 338 874 A1**
(43) Date of publication of application: **29.06.2011**
(21) Application number: 09179438.8
(22) Date of filing: 16.12.2009
(51) Int. Cl.: C07C 227/16, C07C 227/40, C07C 229/76

(54) **Process for the preparation of chelated compounds**

(71) Applicant: Bracco Imaging S.p.A, 20134 Milano (IT)
(72) Inventor: Maisano, Federico, 20097, San Donato Milanese (IT); Crivellin, Federico, 20097, San Donato Milanese (IT)
(74) Representative: Macchetta, Francesco

(57) **Abstract**

The present invention generally relies on a process for the preparation of chelated compounds, comprising the selective interaction between a solid matrix and a chelating agent. In more details, the present invention enables the preparation of chelated compounds useful as diagnostic or therapeutic agents either by a batch or a column procedure, in high yields and in a reliable way.

## Description

The present invention generally relates to a process for the preparation of chelated compounds, comprising the selective ion exchange interaction between a solid matrix and a chelating agent. In more details, the present invention enables the preparation of chelated compounds useful as diagnostic or therapeutic agents.

### Background

Contrast agents (or contrast media) are a class of compounds currently employed in various medical imaging techniques to enhance the contrast of structures or fluids within the body.

From a chemical point of view, contrast agents are characterized by chemical features, which basically depend on the imaging technique they are intended for. It is known in the art that, in Magnetic Resonance Imaging (MRI) and in nuclear medicine techniques, both the contrast and the therapeutic agents are usually compounds constituted by a suitable metal ion, chelated by an appropriate chelating agent, so to form a chelated compound (also indicated as paramagnetic complex, in MRI). To this extent, the common procedures known in the art for the preparation of said complexes foresee the reaction of a chelating agent, usually a polyamino carboxylic acid derivative, with a given metal derivative (e.g. a paramagnetic or a lanthanide metal derivative, or even a radioisotope thereof), in a suitable medium. The chelating agent may be either purchased and used as such, or eventually it may be functionalized or even totally synthesised, for instance, according to procedures known in the art (see among others, WO2007/031390, Bracco).

EP 0230893 discloses the preparation of a series of chelated compounds by reaction of several chelating agents with metal chlorides, in water. In spite of the good yields and reproducibility of the disclosed process the final purification steps are necessary in order to remove the residual salts formed during the complexation reaction.

An alternative to this procedure may be represented, for instance, by the reaction of a chelating agent with a metal oxide in lieu of the metal halide, in order to avoid the formation of the afore mentioned salts as side products.

See, for example, EP 0434345, where a paramagnetic complex is prepared by reaction of a tetraazacyclododecane derivative with Gd₂0₃ in an aqueous solvent system. This methodology however suffers of the problem represented by the low solubility of the starting metal oxide in the reaction medium. Therefore, particular cares have to be devoted to overcome this major issue, such as, e.g., vigorous stirring and high temperatures, with the consequent risk that product degradation or secondary unwanted reactions may occur.

Lever et al. in Nuclear Medicine & Biology, Vol. 23 pp1013-1017 (1996) describe the labelling of a chelating drug by adsorbing radioactive lead ²⁰³Pb) on a Chelex resin, followed by the chelation of the metal by contacting the resin with the chelating drug, i.e., dimercaptosuccinic acid (DMSA) or dibromosuccinic acid (DBSA) . However, when the process is carried out using a column method a large excess of chelating agent is necessarily eluted in order to uptake the metal from the resin, with the consequence that a large amount of non-complexed acid is recovered in the final solution along with the radiolabeled complex. On the other hand, in a batch method, the described process occurred with even lower yields.

From all the above it will be apparent that there is still the need of a convenient and generally applicable way, also on industrial scale, for the preparation of chelated compounds in a pure form, in order to avoid the aforementioned drawbacks.

We have now found that when a metal of choice is sorbed on a solid matrix and an amino carboxylic chelating agent is contacted with said loaded matrix, a corresponding metal chelated compound may be selectively formed, in high yields and in a form substantially free of side products or unreacted material, in a reliable and safe manner.

### Summary of the invention

The present invention therefore generally relies on a process for the preparation of an amino carboxylic chelated compound, comprising the steps of:
a. contacting a liquid composition containing a metal ion component with a solid matrix, to form a metal chelated matrix; and
b. contacting said metal chelated matrix with a liquid composition comprising an amino carboxylic chelating agent.

Advantageously, the present process allows for the recovering of the final complex in high yields and in a substantially pure form, i.e. basically with undetectable amounts of side products or unreacted materials, such as the free chelating agent, or the free metal.

Therefore, the process of the invention may be advantageously employed for the synthesis, even on a large scale, of paramagnetic or radioactive chelated compounds, intended for the use as diagnostic or radio-therapeutic agents.

The metal ion may be selected depending on the final use to which the complex is intended for, and the chelating agent is an amino carboxylic acid derivative, either cyclic or acyclic, or a pharmaceutical acceptable salt thereof.

The solid matrix may be chosen among those which are able to reversibly chelate the metal ion of interest and it may be advantageously employed either in batch or in column procedures.

### Detailed description of the invention

Unless otherwise provided, with the term "chelating agent" (also indicated as "chelating moiety" or "ligand" or "chelator") we intend chemical moieties, agents, compounds or molecules, either *per* se or in the form of a salt thereof, able to form a complex containing at least one coordinated bond with a metal.

Accordingly, and in line with the general definition known in the art, with the terms "chelated compound", "chelated complex" or "complex" we mean a compound consisting of a metal ion connected to a chelating agent.

The expression "solid matrix" or "solid support" or "resin" is intended to include any kind of ion exchange support.

Thus, the term "contained in (or adsorbed on) a solid matrix" is used to indicate that the metal ion is connected to the solid matrix, generally by complexation with a chelating moiety of the matrix itself, so to constitute a metal loaded solid matrix. As mentioned before, the chelated compounds of the present invention are selectively obtained by contacting a liquid composition containing the amino carboxylic chelating agent of choice, with a solid matrix loaded with a selected metal ion. The desired complex is obtained by an ion metal exchange between the solid matrix and the chelating agent and it may be collected in high yields in a substantially pure form, i.e. with undetectable amounts of side products or unreacted materials, such as the unreacted chelating agent or the free metal.

In more details, the solid support should be able to reversibly bind a given metal ion present in a liquid medium, thereby removing or exchanging it from the liquid which the support is contacted with. Such a contact may be realized, for instance, by mixing the matrix together with the mentioned liquid medium or by the elution of the latter through a column containing the matrix. Preferred solid matrix are, for example, those complexing cation exchange resins derivatized with iminodiacetic acid functionalities.

Non limitative examples of said resins are, among others, Amberlite IRC 748 I (Rohm and Haas Company, Philadelphia, U.S.A.), Purolite S-930 (The Purolite Company, Bala Cynwyd, PA, USA), Lewatit TP207 and Lewatit TP 208 (Lanxess AG, Leverkusen, Germany), Chelex 100 (Bio-Rad Laboratories, Richmond, CA, USA).

According to a preferred embodiment of the invention, the solid matrix as such or eventually slurried, for example, in water, is applied to a column, such as for instance a chromatographic column among those commonly used in organic synthesis. A commercially available resin pre-packed column may be suitably employed as well. Before its use, the resin (or the pre-packed column) may be optionally treated to remove eventual impurities that may leach from the column and collect in the eluant, by using procedures known in the art, such as, e.g., multiple water washings.

The column is thus treated, for example, by gravity or by modified pressure elution, or by a suitable pump, with a first liquid medium containing a metal ion component, for a proper frame of time and at a monitored flow rate. These conditions should enable the matrix to reversibly adsorbs the metal, typically by complexation, so to constitute a metal loaded solid matrix.

Unless stated otherwise, "proper frame of time" means that the elution (or in case multiple elutions carried out by recycling the same eluted medium) through the column occurs until a desired percent of the matrix, preferably about 70-100% of the theoretical capacity of the resin (according to the resin description leaflets), is chelated with the metal ion. The actual amount of the loaded metal depends also on the elution conditions and can be monitored by measuring the remaining metal concentration in the eluate and calculating by difference the total molar amount of the loaded metal.

Typical elution times lie between about 30 minutes to about 3 hours. "Monitored flow rate" indicates a flow rate, generally a constant flow rate, of about 1-50 bed-volume/h set for instance according to the selected matrix, or to the concentration or the viscosity of the liquid composition containing the metal ion of choice.

In this respect, any suitable metal ion component may be conveniently used, whereas inorganic salts such as halide (e.g. chloride, bromide and the like), or organic salt, such as e.g. acetate and the like, are preferred, either in an anhydrous or hydrate form.

The metal ion is selected from the group consisting of: transition metal ions, lanthanide metal ions, aluminium(III), gallium(III), indium(III), tin(II), and lead(II). According to the general knowledge, transition metals and lanthanide metals are those metallic elements with atomic number ranging from 21 to 30, from 39 to 48, from 57 to 80 and from 89 to 103.

In a preferred embodiment of the invention, the metal ion is gadolinium(III), and the metal salt is gadolinium chloride (GdC1₃) or gadolinium acetate (Gd(OAc)₃). Furthermore and according to another embodiment of the present invention, the metal may be selected among the commonly employed radioisotope atoms, such as for example ⁶⁴Cu, ¹¹¹In, ^{99m}TC, ¹⁵³Gd, ⁹⁰Y, ¹⁶⁶Ho ¹⁸⁶Re, and ¹⁷⁷Lu.

The resin is loaded, or even saturated, with the chosen metal ion component, the latter typically being present in a liquid medium, such as e.g. an aqueous system. In particular, the metal component may be dissolved or suspended (at room temperature or at higher temperatures) for instance, in water, distilled water, pyrogens free water, water for injection (also indicated as WFI) and the like; as well as in any aqueous mixture with a polar organic solvent. Non limiting examples of such polar organic solvents are low (C₁-C₄) alcohols, tetrahydrofuran (THF), and the like, including mixtures thereof.

Preferably, the metal ion component is solved in the liquid medium of choice.

The metal salt solution should preferably have a pH value in the range of about 3-8, and in case, the pH may be adjusted by addition of a proper amount of a base, such as, for example, an alkaline base or the like.

Once the metal has been adsorbed as previously indicated, the resin is preferably washed with an aqueous liquid medium to remove excess metal and eventually some loosely bound metal ions. To this end, selected anions may be included in said liquid medium, such as, but not limited to, anions of small organic acids, e.g., acetate, propionate, succinate, citrate, etc.

After this washing step, a liquid composition containing the amino carboxylic chelating agent is contacted with the loaded matrix, typically by percolation through the column under monitored conditions, for a period of time of about 10 to 180 min, or alternatively, by mixing of the chelating agent solution and the resin together in a batch procedure.

A suitable chelating agent may be represented, for instance, by an amino carboxylic compound which is able to selectively remove the metal ion adsorbed on the matrix. In this direction, general examples of chelating agents are either cyclic or acyclic amino carboxylic acids selected from: BOPTA (4-carboxy-5,8,11-tris(carboxymethyl1)-1-phenyl-2-oxa-5,8,11-triazatridecan-13-oic acid), DTPA (diethylene triamine pentaacetic acid), DOTA (3,6-dioxaoctane-1,8-diamine-NNN'N'-tertaacetic acid), EDTA (ethylenediamine-tetraacetic acid), HEDTA (N-hydroxyethyl-ethylenediamine triacetic acid), CDTA (1,2-cyclohexylendinitro-tetraacetic acid), NTA (nitrilotriacetic acid) and the like; whereas pentacarboxylic acid derivatives like BOPTA and DTPA are the more preferred ones.

Practically, and by analogy to what previously described, the chelating agent is dissolved or suspended in a liquid medium selected among those as defined above (i.e. water, aqueous mixture of polar organic solvents, WFI and the like), so to constitute a chelating agent liquid medium.

Preferably, the chelating agent is solved in the liquid medium.

Optionally, in order for instance to increase the solubility of the chelating agent, an inorganic base such as NaOH, Na₂CO₃ and the like, or an organic base, such as ammonia, meglumine and the like, may be added to the solution, generally in a stoichiometry ratio of 1 to 5 equivalents, with respect to the chelating agent, forming by that the corresponding chelating agent salt thereof. Preferably, the base is meglumine and the molar ratio is 2:1 with respect to the chelating agent.

The concentration of the solution may be properly chosen, for example, so that an efficient and convenient elution through a column may be obtained, or, in case of a batch procedure, a convenient mixing with the resin occurs.

It will be self evident to the skilled in the art that the molar amount of the chelating agent in solution should not be in excess over the metal amount actually present on the matrix, in order to avoid the elution of the chelating agent in excess together with the formed complex.

The process may be monitored by using common analytical methods, i.e. for instance, UV-detection or thin layer chromatography (TLC) or complexometric titrations.

As indicated in the experimental part, in a typical column procedure of the present invention, the chelated complex may be conveniently collected in high yields in a pure form and with a content of impurities lower than 0.5%.

For instance and according to a preferred embodiment, the BOPTA ligand is solved in water for injection (WFI) containing 2 equivalents of meglumine, at room temperature, forming a BOPTA-meglumine salt 1:2 solution, with a concentration of 0.1 M, which is then eluted through a column containing the Amberlite IRC 748i resin saturated with Gd(III), at an elution rate of about 1 to 1.5 bed-volume/h. The desired meglumine salified Gd-BOPTA complex is selectively obtained in high yield (> 90%, purity >99.5%) with undetectable amount of free ligand or other side products. Eventually, the thus obtained complex may be neutralized with additional meglumine, giving by that a solution containing the gadobenate dimeglumine compound of formula I:

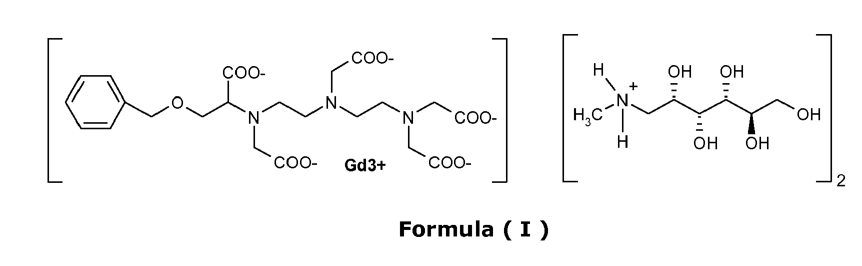

The chelated agents obtained by the present process may be used, for example, in the preparation of injectable formulations complying with the requirements and the guidelines demanded by the various regulatory authorities. For example, the concentration of the chelated agent solution may be adjusted to proper values by evaporation, nanofiltration or addition, for instance, of WFI (or any another proper medium) and the addition of supplementary pharmaceutical ingredients (i.e. adjuvants, stabilizers, carriers, pharmaceutical acceptable neutralizing agents and the like) may be required before submitting the composition to a final sterilization step. Likewise, this final step may be performed by procedures commonly employed in the art, for instance by autoclaving.

Accordingly, the gadobenate dimeglumine solution obtained as previously described, may be used, fro example, in the preparation of the commercially available contrast agent formulation, commonly known as MuItiHance®. Alternatively, the chelated compounds of the present process, may be isolated in a solid form, by means of procedures known in the art, such as, for instance, evaporation of the solvent, liophilization, spray-drying, and the like. Said solid form may be conveniently stored or employed, for example as a part of a kit, intended for diagnostic or therapeutic purposes.

From all the above, it will be apparent that the process of the present invention advantageously enables the preparation of various chelated compounds, by an efficient and time saving procedure, substantially avoiding the drawbacks related to the prior-art procedures. As extensively reported, said chelated complexes may be employed in the preparation of diagnostic or radiotherapeutic agents, mainly depending on the nature of the metal of choice.

It should be also understood that the particular embodiments illustrating the present invention are exemplary only and not to be regarded as a limitation of the present invention.

The following examples of the practice of the present invention are meant to be illustrative and are in no way limiting the scope of the invention.

### EXPERIMENTAL PART

### Example 1: Loadinci the resin with metal ions

A 10 x 90 mm column was prepared with 5 g of Amberlite IRC748I (Rohm and Haas Company, Philadelphia, U.S.A.) in water column. A 10-100 mM Gadolinium Acetate hydrate (325678, Aldrich) solution (minimum content 0.75 mmol Gd) was circulated through the column for 3 hours at 5 mL/min. Then, loosely bound metal ions were removed by washing the column with 30 mL of a 0.5-2 M Sodium Acetate Solution pH 6, followed by water until no more Gadolinium was in the flow through. The absence of Gadolinium was proved by dropping 100 µL of column flow through into 400 µL of Xylenol Orange solution (Xylenol Orange 0.005% in 0.2 M MES buffer pH 5.8): it should not turn violet.

### Example 2: Complex preparation - column procedure

A 86 mM BOPTA dimeglumine solution (7.5 mL) was loaded on the column prepared as described in Example 1 and eluted with water at flow rate 0.2 mL/min. The run was monitored for UV absorbance, pH and conductivity. 1-mL fractions were collected.

The gadobenate (UV absorbing) containing fractions were pooled together (11 mL). Gadobenate concentration was determined by HPLC analysis: 56 mM, accounting for 96% yield. No free Gd or free BOPTA were detectable by complexometric titrations.

### Example 3: Complex preparation - batch procedure

The resin prepared as described in Example 1 was transferred into a 15 mL polypropylene test tube, with the addition of 4 mL water and 7.5 mL of 88 mM BOPTA dimeglumine. The resin was gently shacked for 1 h at room temperature. At the end of the incubation the resin was transferred again in the column and the solution was recovered with a syringe. Water was added to the resin (2 x 7 mL), withdrawn with the syringe and combined with the previous Gadobenate solution. Yield: 23.5 mL of 26 mM Gadobenate, accounting for 93% yield. No free Gd or free BOPTA were detectable by complexometric titrations.

## Claims

1. A process for the preparation of a metal chelated compound or a salt thereof, comprising the steps of:
a. contacting a liquid composition containing a metal ion component with a matrix, to form a metal chelated matrix; and
b. contacting said metal chelated matrix with a liquid composition containing an amino carboxylic chelating agent or a salt thereof.

2. A process according to claim 1 wherein the metal ion component is a metal salt or a metal oxide.

3. A process according to claim 1 step a, and to claim 2 wherein the metal ion is selected from the group consisting of: transition metal ions, lanthanide ions, aluminium(III), gallium(III), indium(III), tin(II), lead(II).

4. A process according to claim 1-3 wherein the metal ion is gadolinium (III).

5. A process according to claims 1-4 wherein the metal ion component is gadolinium acetate.

6. A process according to claim 1-5 wherein the metal ion is a radioisotope ion.

7. A process according to claim 1 wherein the liquid composition in step a and in step b is an aqueous solution or an aqueous suspension.

8. A process according to claim 7 wherein the liquid composition is WFI.

9. A process according to claim 1 wherein in step a and b said matrix is an ion exchange resin.

10. A process according to claim 1 and 9 wherein the matrix contains iminodiacetate functional groups.

11. A process according to claim 1, wherein the chelating agent of step b is BOPTA or a pharmaceutical acceptable salt thereof.

12. A process according to claim 1 for the preparation of gadobenate dimeglumine.
